# EUROPÄISCHE PATENTANMELDUNG

(11) **EP 1 382 728 A1**
(43) Veröffentlichungstag der Anmeldung: **21.01.2004**
(21) Anmeldenummer: 03015610.3
(22) Anmeldetag: 15.07.2003
(51) Int. Cl.: D04B 21/20

(54) **Implantierbares textiles Flächengebilde**

(30) Priorität: 15.07.2002 DE 10231975
(71) Anmelder: Serag-Wiessner KG, 95119 Naila (DE)
(72) Erfinder: Gramalla, Oliver, 95152 Selbitz (DE)
(74) Vertreter: Klingseisen, Franz, Dipl.-Ing.

(57) **Zusammenfassung**

Zur Ausbildung eines implantierbaren bandförmigen Flächengebildes, das ohne schlauchförmige Hülle in Körpergewebe eingezogen werden kann, werden längs der Ränder des Bandes seitlich abstehende Schlaufen (3) vorgesehen, die bei einer vorgegebenen Zugkraft an dem Band nach innen umklappen. Der die Schlaufen (3) bildende Faden (4) ist lose längs der Ränder in das Flächengebilde eingezogen.

## Beschreibung

Die Erfindung betrifft ein implantierbares textiles Flächengebilde für verschiedene medizinische Einsatzgebiete, insbesondere für die Harninkontinenzbehandlung bei Frauen.

DE 101 07 521 Al beschreibt ein zugstabiles elastisches Band für die operative Behandlung der Harninkontinenz der Frau, wobei das Band an seinen Längskanten Schlaufen aufweist, die fest in die Struktur des Bandes eingebunden sind. Bei einer Operation würden diese seitlich abstehenden Schlaufen das Durchziehen des Bandes durch das Körpergewebe behindern und zu einer zusätzlichen Traumatisierung des Körpergewebes führen. Daher wird das Band zum Einziehen in das Körpergewebe mit einer schlauchförmigen Hülle umschlossen, die nach Plazieren des Bandes leicht entfernt werden kann.

Der Erfindung liegt die Aufgabe zugrunde, ein derartiges Band mit seitlich abstehenden Schlaufen so auszubilden, dass es ohne schlauchförmige Hülle in das Körpergewebe eingezogen werden kann und dennoch zu keiner Traumatisierung des Körpergewebes führt.

Dies wird erfindungsgemäß dadurch erreicht, dass die Schlaufen derart ausgebildet sind, dass bei einer gewissen Zugkraft an dem textilen Band die Schlaufen nach innen klappen und das Einziehen in das Körpergewebe nicht behindern.

Beispielsweise Ausführungsformen der Erfindung werden nachfolgend anhand der Zeichnung näher erläutert. Es zeigen
- Fig. 1: in einer Draufsicht ein bandförmiges textiles Flächengebilde mit Schlaufen,
- Fig. 2: ein Schemabild der Bindungstechnik des Bandes nach Fig. 1,
- Fig. 3: eine deutlichere Darstellung des Schussfadenverlaufs eines Bandes nach Fig. 1 ohne Maschenstränge,
- Fig. 4: in schematischer Darstellung verschiedene Zustände des Bandes durch Ziehen an dem die Schlaufen bildenden Faden, und
- Fig. 5: eine graphische Darstellung der Schlaufenumklappkraft über die Länge des Bandes.

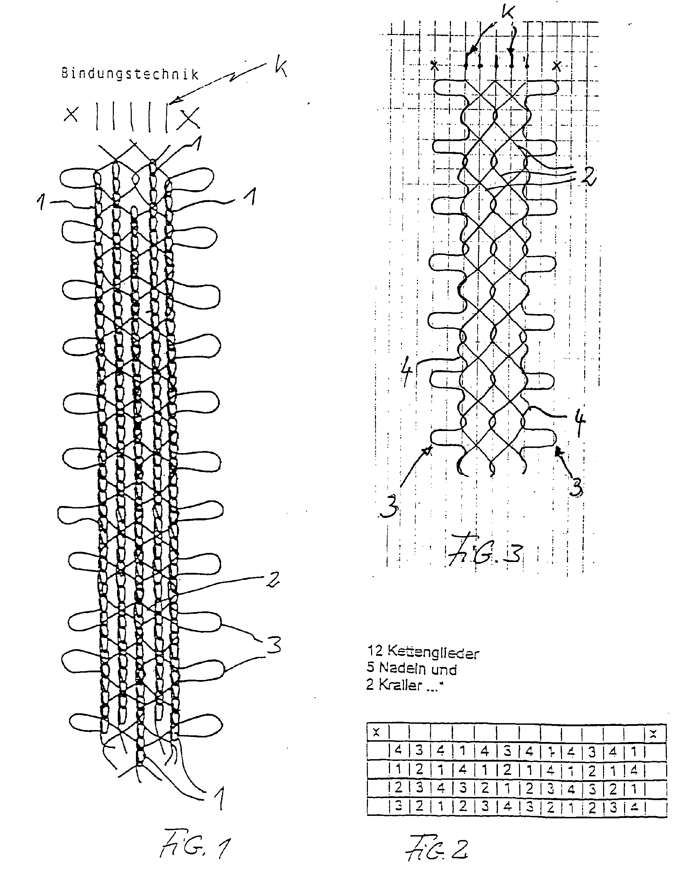

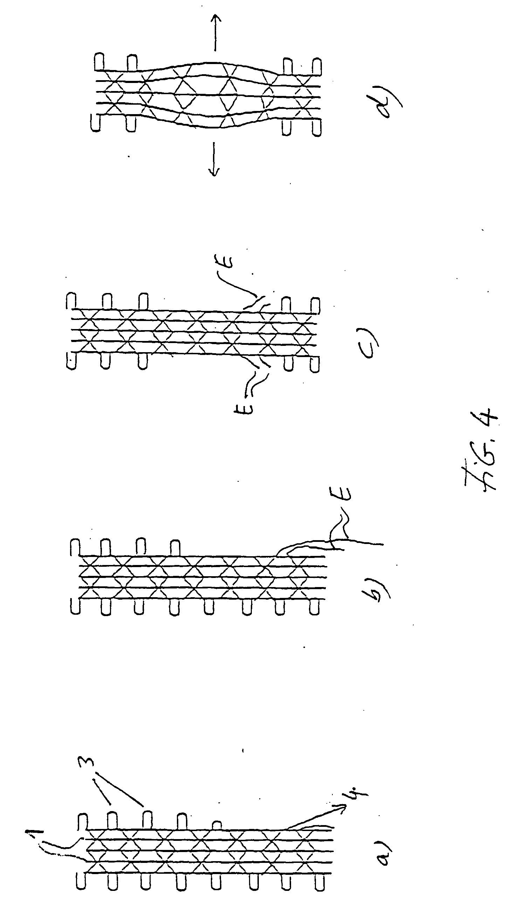

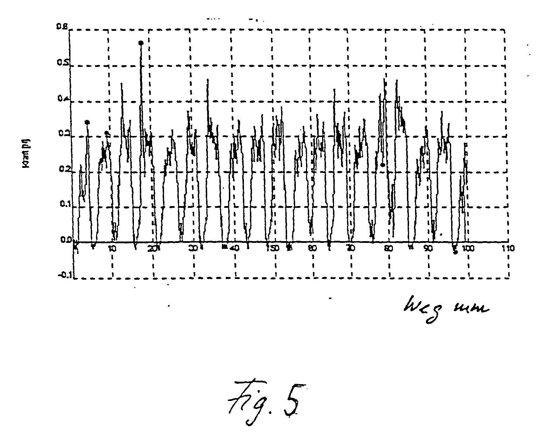

Fig. 1 zeigt eine Ansicht eines bandförmigen textilen Flächengebildes, das aus einer Struktur aus Schussfäden 2, wie sie in Fig. 3 ohne Kette wiedergegeben ist, und fünf Strängen 1 aus Maschenware aufgebaut ist, die den Grundkörper bzw. die Kette bilden. Diese Stränge 1 verlaufen längs den in den Fig. 1 und 3 mit K bezeichneten Linien. Die einzelnen, beabstandeten Stränge 1 der Kette sind mit den Schussfäden 2 lose verbunden und legen die Gestalt des Flächengebildes fest, indem sie die Schussfäden in der in Fig. 3 wiedergegebenen Form zusammenhalten. An den Rändern dieses bandförmigen Flächengebildes sind seitlich abstehende Schlaufen 3 ausgebildet.

Die Schlaufen 3 werden durch ein gegenüber dem Aufbau des bandförmigen Flächengebildes unabhängiges Monofilament 4 ausgebildet, das nur längs der beiden Ränder in den textilen Aufbau des Flächengebildes eingezogen ist, wie sich dies aus dem Schemabild der Bindungstechnik nach Fig. 2 ergibt. Dadurch, dass die Schlaufen 3 durch ein gesondertes, längs des Randes eingezogenes Monofilament 4 ausgebildet sind, können die Schlaufen verändert bzw. glatt gezogen werden, wie dies anhand von Fig. 4 näher erläutert wird, ohne dass das aus den Strängen 1 und der Schusslegung 2 bestehende bandförmige Flächengebilde selbst beeinträchtigt oder verändert wird. Das Monofilament 4 wird durch die äußere Kette bzw. den äußeren Strang 1 lose im Verbund des Flächengebildes gehalten.

Das in Fig. 1 und 3 wiedergegebene bandförmige Flächengebilde wird auf einer Häkelgalonmaschine mit Kralleneinrichtung in der Sechserteilung hergestellt, wobei in Kette und Schuss ein Monofilament verwendet wird. In die als Franse ausgebildete Kette 1 (Fig. 1) wird die Schusslegung, wie sie Fig. 3 zeigt, eingebracht, die bei jeder vierten Maschenreihe von einer Kralleneinrichtung als Schlaufe 3 beidseitig ausgebildet wird. Die Schussfäden 2 bilden zwei gegenläufige Schusslegungen, die ebenfalls in die Franse bzw. Kette 1 eingebunden werden. Die Schlaufen 3 am Rand werden somit durch einen gesonderten Schussfaden 4 erzeugt. Die Stränge 1 können beispielsweise mit sechs Maschen pro Zentimeter gearbeitet werden. Die Länge der abstehenden Schlaufen 3 beträgt bei dem Ausführungsbeispiel in Fig. 1 etwa 4 mm.

Insgesamt sind bei dem Flächengebilde nach Fig. 1 als Kette fünf Fransenstränge 1 und sechs Schussfadensysteme vorhanden, zwei für den schlaufenbildenden Faden 4 und vier für den Bereich zwischen den Fransensträngen. Diese binden die Schussfäden nicht fest ein, sondern umschlingen diese nur lose, so dass die Fransenstränge 1 auf den quer liegenden Schussfäden hin- und hergeschoben werden können, wie sich dies auch aus Fig. 4 ergibt. Jeder einzelne Schussfaden 2 und auch die die Schlaufen 3 bildenden Schussfäden 4 sind so in die Kette eingebunden, dass jeder einzelne Schussfaden herausgezogen werden kann.

Das Band ist so gefertigt, dass ab einer gewissen Zugbeanspruchung die monofilen Schlaufen 3 in Verbindung mit der Reibung am Körpergewebe nach innen umklappen. Beim Durchzug des Bandes durch das menschliche Gewebe muss diese Zugkraft beibehalten werden. Lässt diese Kraft nach oder wird keine Zugkraft mehr ausgeübt, so klappen die monofilen Schlaufen 3 wieder nach außen und dienen zur Verankerung des Bandes im Körpergewebe. Es ist für den korrekten Sitz des implantierten Bandes wichtig, dass die für das Umklappen der Schlaufen 3 nötige Kraft größer ist als die Kraft, die bei Bewegungen der Patientin auftreten. Nachdem sich die Patientin in einem Zeitraum von 4 - 6 Wochen schonen und auf anstrengende Aktivitäten verzichten soll, wird davon ausgegangen, dass im Körper der Patientin Kräfte von deutlich weniger als 12 N wirken, wobei 12 N der maximalen Kraft entspricht, die im Bauchraum auftreten kann. Die Untergrenze für die zum Umklappen der Schlaufen erforderliche Kraft wird somit auf etwa 12 N ausgelegt.

Fig. 5 zeigt den Kraftverlauf zum Umklappen der Schlaufen 3 beim Einziehen in Körpergewebe.

Um ein Umklappen der Schlaufen bei einer vorbestimmten Zugbeanspruchung in Verbindung mit der Reibung des Bandes am Körpergewebe, in das das Band eingezogen wird, zu erreichen, können in dem die Schlaufen 3 bildenden Monofilament bzw. Schussfaden 4 Schwachstellen vorgesehen werden, die ein Umklappen der Schlaufen an den Schwachstellen begünstigen.

Wenn keine Schwachstellen vorgesehen werden, kann das Umklappen der Schlaufen durch Wahl der Stärke, der Materialart und der Biegesteifigkeit des Monofilaments 4 beeinflusst werden. Auch die Länge und Breite der Schlaufen beeinflussen das Verhalten beim Einziehen des Bandes in das Körpergewebe bei einer bestimmten Zugkraft.

Bei einem Ausführungsbeispiel eines Bandes nach Fig. 1 haben die monofilen Schlaufen 3 eine Breite von 1,5 mm und eine Abstehlänge von 3,5 mm. Das Schlaufenmaterial besteht aus einem monofilen Polypropylenfaden der Stärke EP 1,5 mit einer Biegesteifigkeit von 2 mg (Werte mit Gurly-Stiffness-Tester ermittelt). Zum Vergleich klappt ein Faden mit der Stärke EP 1 und einer Biegesteifigkeit von 0,5 mg zu leicht um und steht nicht wieder auf, während ein Faden mit der Stärke EP 2 und einer Biegesteifigkeit von 5 mg nicht umklappt und beim Einziehen in das Körpergewebe zu Gewebebeschädigungen führt.

Bei diesem Ausführungsbeispiel wird beim Durchzug des Bandes durch das Körpergewebe eine durchschnittliche Kraft von 0,15 N pro Schlaufenpaar benötigt. Wird das Band einseitig durch das Körpergewebe gezogen (Implantationslänge links und rechts der Harnröhre von jeweils ca. 10 cm), so kommen 20 Schlaufenpaare zum Einsatz, die mit einer durchschnittlichen Kraft von 3 N (20 x 0,15 N) umgeklappt werden müssen. Werden vom Arzt diese 3 N überwunden, so kann das Band durch den Körper gezogen werden, während die Schlaufen nach innen umgeklappt sind. Lässt diese Kraft nach, beispielsweise wenn die Implantationslage erreicht ist, so stellt die Rückstellkraft des Filaments 4 die Schlaufen 3 wieder auf. Hierbei ist eine Rückstellkraft von über 0,3 N an jeder Schlaufe erforderlich. Anders als bei der Durchzugsbeanspruchung wirken in der Ruhelage des Bandes die Maximalkräfte gegen das Körpergewebe, weswegen eine höhere Rückstellkraft erforderlich ist, um das Schlaufenpaar zurückzuklappen.

Bei einer durchschnittlichen Implantationslänge von 2 x 10 cm und einer Anzahl von 20 Schlaufenpaaren pro 10 cm ergibt sich insgesamt eine Haltekraft von 12 N (40 x 0,3 N). Diese entspricht der vorgegebenen Grenze von 12 N.

Durch den beschriebenen Schlaufenklappeffekt ist es möglich, das Band ohne zusätzliche Schutzhülle in das Körpergewebe einzuziehen. Hierbei kommt es auch auf die Stabilität des Bandes selbst an. Es muss gewährleistet werden, dass das Band in seiner Flächenausdehnung stabil bleibt. Es darf sich nicht unter Zugbeanspruchung zu einem Schal zusammenziehen, wie dies bei Netzen häufig der Fall ist. Auch beim Einwachsen im Körpergewebe kann es in der Praxis dazu kommen, dass durch den Einsprossvorgang mit Fibroblasten ein textiles Band weiter gedehnt wird. Der beschriebene Aufbau des Bandes dagegen lässt solche Verschiebungen nicht zu.

Die Stabilität des Bandes wird durch den Grundkörper aus den Kettsträngen 1 aus Maschenware längs den Linien K erreicht, die durch Schussfäden 2 längs der Linien K zusammengehalten werden. Das Band nach Fig. 1 hat somit in Längsrichtung einen stabilen Aufbau, der sich unter Zugbeanspruchung nicht zusammenzieht.

Das Zusammenspiel des vollflächig stabilen Bandaufbaus und der umklappbaren Schlaufen 3 macht es möglich, dass beim Einziehen des Bandes in Körpergewebe eine Schutzhülle entfallen kann. Hierdurch ist es möglich, das Band auch noch nachträglich in seiner Position zu korrigieren, ohne den sicheren Halt und Sitz im Körpergewebe zu gefährden. Hierbei muss der Arzt nur einmal die Haltekraft des Bandes durch Zug überwinden und damit die Schlaufen wieder umklappen, worauf das Band in die optimierte Lage gebracht werden kann. Nachdem keine Zugkraft mehr anliegt, werden die Schlaufen erneut nach außen geklappt.

Ein weiterer Vorteil des beschriebenen Bandaufbaus ist, dass der Operateur das Band auch noch während der Operation individuell an die anatomischen Strukturen der Patientin anpassen kann. Dadurch, dass die Schlaufen 3 durch das längs des Randes des Bandes eingebrachte Monofilament 4 nicht fest in den Bandaufbau eingebunden sind, können durch Ziehen an dem die Schlaufen bildenden Filament 4 einzelne Schlaufen glatt gezogen werden, wie dies Fig. 4a zeigt. Deshalb ist es auch möglich, die Schlaufen 3 in bestimmten Bereichen zu entfernen. Dazu zieht der Operateur an einer Schlaufe und entfernt dadurch den Schlaufenfaden an den erforderlichen Bereichen an dem Band, wie dies Fig. 4a - 4c zeigen. Der Schlaufenfaden 4 wird zum Glattziehen von Schlaufen 3 durchtrennt, so dass zwei Enden E aus dem Bandaufbau herausragen, wie dies Fig. 4b zeigt. Diese Enden E werden bis auf eine Länge von 4 bis 8 mm abgetrennt, wie Fig. 4c zeigt.

Die auf eine verbleibende Länge von etwa 4 bis 8 mm abgeschnittenen Enden E der Schlaufenfäden 4 bewirkt, dass sich die Wirkstruktur nicht auflöst. Wird das Band durch seitlichen Zug an den Fransensträngen 1 im Bereich der eingezogenen Schlaufen verbreitert, so ziehen sich diese abstehenden Fadenenden E in den Bandaufbau zurück, wie Fig. 4d zeigt.

Die Breite des Bandes selbst kann durch Zug an den Seiten individuell angepasst werden, wie dies in Fig. 4d wiedergegeben ist. Hierdurch ist es möglich, z. B. unterhalb der Harnröhre die Ankerschlaufen zu entfernen und die Unterlagefläche zu verbreitern. Gerade in diesem empfindlichen Körpergewebe um die Harnröhre bringt dies deutliche Vorteile gegenüber einem herkömmlichen starren Bandaufbau.

Die offenen Maschen der einzelnen Stränge 1 und die Zwischenräume zwischen den Strängen 1 begünstigen das Einsprossen von Körpergewebe nach der Implantation. Die längs des Randes des bandförmigen Flächengebildes beabstandeten Schlaufen 3 bilden einen sauberen Abschluss am Rand des Flächengebildes und begünstigen die Adaption im Körpergewebe, da die Schlaufen durch ihre Rundungen das Körpergewebe nicht irritieren. Zugleich dienen diese Schlaufen 3 für eine gute Verankerung des Flächengebildes im Körpergewebe.

Die Breite des bandförmigen Flächengebildes kann unterschiedlich ausgelegt sein entsprechend den jeweiligen Operationsverfahren und Operationstechniken. So wird z. B. für den Einsatz als Band zur Unterstützung des Körpergewebes im Beckenboden ein ca. 1 cm breites Band zur Harninkontinenzbehandlung vorgesehen.

Bei herkömmlichen netzförmigen Flächengebilden ergibt sich unter Zugbeanspruchung ein so genannter Schal, d. h. die Maschenware rollt sich unkontrolliert zusammen, so dass es zu irreversiblen Verdrillungen kommt und das textile Flächengebilde nur schwer durch das Körpergewebe gezogen werden kann. Durch den beschriebenen Aufbau des bandförmigen Flächengebildes bleibt dieses bei Zugbeanspruchung flach. Lediglich die seitlich abstehenden Schlaufen klappen je nach Zugbeanspruchung um. Das Band kann somit problemlos, sicher und gewebeschonend in die richtige Lage im Körper eingezogen werden. Sobald sich das Band in der richtigen Lage befindet und entspannt wird, klappen die Schlaufen 3 wieder nach außen und gewährleisten eine gute Fixierung in körpereigenen Gewebeschichten.

Die einzelnen Stränge 1 können aus Monofilamenten bestehen. Nach einer anderen Ausführungsform können die Stränge 1 aus einem Multifilament ausgebildet sein. Der Aufbau der Stränge 1 aus einem Multifilament wird von Patienten als angenehmer bewertet und setzt die Heilungsdauer herab. Die Schussfäden 2 sind in beiden Fällen ein Monofilament.

Bei beiden Ausführungsformen ergibt sich durch die Stränge 1 aus Maschenware ein stabiles Grundsystem mit großen Porenöffnungen, während der Textilabschluss ohne traumatisierende Ränder ausgestaltet ist. Die monofilen Schlaufen 3 begünstigen in beiden Fällen einen optimalen Durchzug durch das Körpergewebe und eine gute Fixierung im Körper.

Anstelle des beschriebenen Aufbaus des bandförmigen textilen Flächengebildes kann auch ein Gewebe, Gelege oder ein Vlies vorgesehen sein, an dessen Rändern durch einen gesonderten Faden die Schlaufen 3 ausgebildet sind. Es wird unabhängig vom Aufbau des Flächengebildes der Vorteil erzielt, dass das Band durch die seitlich abstehenden und umklappbaren Schlaufen einen leichten und schonenden Gewebedurchtritt ermöglicht und das implantierte Band vom körpereigenen Gewebe gut adaptiert wird.

Das beschriebene bandförmige Flächengebilde ist sterilisierbar und verliert durch die herkömmliche Gas- oder Hitzesterilisation keine seiner Eigenschaften.

Vorzugsweise wird das Band aus einem Material gefertigt, das einen blauen, grünen oder violetten Farbton hat, damit es von rotem oder gelbem Körpergewebe besser unterschieden werden kann, um eine sichere Durchtrennung des Bandes zu ermöglichen, wenn eine Reoperation erforderlich sein sollte.

Nach einer weiteren Ausgestaltung wird ein dreiteiliger Aufbau des Bandes vorgesehen, wobei der Mittelteil aus einem formstabilen Band entsprechend Fig. 1 ausgebildet ist, an dessen Rändern keine Schlaufen vorgesehen sind. Dieser Mittelteil aus den durch die Schussfäden 2 verbundenen Strängen 1 aus Maschenware kann beispielsweise aus Polypropylen bestehen. Die Breite des Mittelteils kann 2 bis 10 cm betragen. An diesem Mittelteil werden an beiden Seiten Schlaufenbänder angebracht, die aus resorbierbarem Material bestehen. Hierdurch wird erreicht, dass das Band nach der Positionierung an der richtigen Stelle korrekt einwächst und später nur so wenig wie nötig Fremdmaterial im Körper der Patientin zurückbleibt. Die Resorptionszeit wird zweckmäßigerweise so gewählt, dass das Polypropylen-Band eingewachsen ist und erst dann die Festigkeit der resorbierbaren Fäden der Schlaufenbänder auf den beiden Seiten deutlich abnimmt. Es verbleibt bei dieser Ausgestaltung ein formstabiles Band als Implantat, das aus den Kettsträngen 1 aus Maschenware und den Schussfäden 2 besteht.

Ein derartiges formstabiles Band mit dem beschriebenen Aufbau, an dessen Rändern keine Schlaufen ausgebildet sind, kann auch für andere Einsatzgebiete vorteilhaft verwendet werden, wenn es beispielsweise nur um das Abstützen eines Bereichs des Körpergewebes geht.

## Patentansprüche

1. Implantierbares textiles Flächengebilde in Bandform mit an den Rändern seitlich abstehenden Schlaufen (3), die aus einem längs des Randes des Bandes eingezogenen Filament (4) geformt und so ausgebildet sind, dass sie bei einer vergebenen Zugkraft am Band nach innen umklappen.

2. Flächengebilde nach Anspruch 1, wobei das Filament (4) zur Ausbildung der Schlaufen (3) verschiebbar an den Rändern eingelagert ist.

3. Flächengebilde nach den Ansprüchen 1 und 2, wobei der Grundaufbau des Bandes aus einem Gewebe, Gelege, aus Maschenware oder einem Vlies besteht.

4. Flächengebilde nach den Ansprüchen 1 und 2, bei dem etwa parallel zueinander und in einem Abstand voneinander verlaufende Stränge (1) aus Maschenware als Kette durch Schussfäden (2) zu einem formstabilen Band verbunden sind.

5. Flächengebilde nach den vorhergehenden Ansprüchen, wobei die Schlaufen (3) aus Monofilament ausgebildet sind.

6. Flächengebilde nach Anspruch 4, wobei die Stränge (1) aus Maschenware aus Monofilament oder Multifilament ausgebildet sind.

7. Flächengebilde nach den vorhergehenden Ansprüchen, wobei ein Mittelteil aus nicht resorbierbarem Material ausgebildet ist, auf dessen beiden Seiten mit Schlaufen (3) versehene Bänder aus resorbierbarem Material angebracht sind.

8. Flächengebilde nach den vorhergehenden Ansprüchen, wobei die Schlaufen (3) beim Einziehen im Körpergewebe bei einer Zugkraft an dem Band von mehr als etwa 12 N umklappen.

9. Flächengebilde nach den vorhergehenden Ansprüchen, wobei der die Schlaufen (3) bildende Faden ein monofiler Polypropylenfaden der Stärke EP 1,5 mit einer Biegesteifigkeit von 2 mg ist.
